# EUROPÄISCHE PATENTANMELDUNG

(11) **EP 0 922 702 A1**
(43) Veröffentlichungstag der Anmeldung: **16.06.1999**
(21) Anmeldenummer: 97121997.7
(22) Anmeldetag: 13.12.1997
(51) Int. Cl.: C07D 295/185, A61K 31/445, C07D 317/22, C07C 229/50, C07C 233/52, C07C 233/91, C07F 9/40

(54) **Neue Azulenderivate und diese enthaltende Arzneimittel**

(71) Anmelder: Roche Diagnostics GmbH, 68298 Mannheim (DE)
(72) Erfinder: Friebe, Walter-Gunar, Dr., Dipl.-Chemiker, 68165 Mannheim (DE); Dickhaut, Joachim, Dr., Dipl.-Chemiker, 69121 Heidelberg (DE); Haag, Rainer, Dr., Dipl.-Chemiker, 68526 Ladenburg (DE); Leinert, Herbert, Dr., Dipl.-Chemiker, 64646 Heppenheim (DE); Grams, Frank, Dr., Dipl.-Chemiker, 68219 Mannheim (DE)

(57) **Zusammenfassung**

Gegenstand der vorliegenden Erfindung sind neue Azulenderivate der allgemeinen Formel I 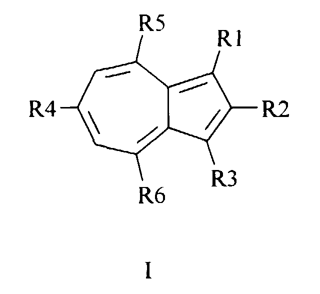 wobei R₁ bis R₆ die in der Beschreibungoben angegebene Bedeutung haben sowie deren Tautomere, Enantiomere, Diastereomere, Racemate und physiologisch vertraglichen Salze oder Ester und Substanzen, die *in vivo* zu Verbindungen der Formel I hydrolysiert oder metabolisiert werden.

Die Erfindung betrifft auch Verfahren zur Herstellung der obigen Verbindungen, Arzneimittel, die solche Verbindungen enthalten, sowie die Verwendung dieser Verbindungen bei der Herstellung von Arzneimitteln mit Metalloprotease-inhibierender Wirkung.

## Beschreibung

Die vorliegende Erfindung betrifft neue Azulenderivate, sowie Arzneimittel, die diese Substanzen enthalten.

Gegenstand der vorliegenden Erfindung sind neue Azulenderivate der allgemeinen Formel I, 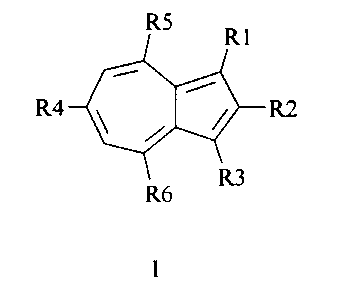 wobei
R₁ und R₃
   einzeln und unabhängig voneinander Wasserstoff Hydroxy, Amino, Mercapto, einen geraden oder verzweigten, gesättigten oder ungesättigten aliphatischen Rest mit 1 - 15 Kohlenstoffatomen, der ein oder mehrfach substituiert sein kann, eine Alkoxy-, Alkylamino-, Dialkylamino-, Alkylmercapto-, Alkylsulfinyl, Alkylsulfonyl-, Alkenyl-, Alkinyl-, Alkenyloxy-, Alkenylmercapto-, Alkinyloxy-, Alkinylmercapto-, Alkylcarbonylamino-, Dialkylcarbonylamino-, Alkylaminocarbonyl-, Formyl-, Alkylcarbonyl-, Carboxyl-, Alkoxycarbonyl-, Alkenyloxycarbonyl-, Alkinyloxycarbonyl-, Carboxyalkyl-, Alkyloxycarbonylalkyl, Alkenyloxycarbonylalkyl-, Alkinyloxycarbonylalkyl-, Nitro-, Cyano-, Halogen-, Trifluormethyl-, oder Azidogruppe, Phenyl oder Phenylcarbonyl, das gegebenenfalls ein- oder mehrfach unabhängig voneinander substituiert sein kann, einen carbocyclischen mono-, bi- oder tricyclischen Rest mit 7-15 C-Atomen oder ein heterocyclisches mono-, bi- oder tricyclisches Ringsystem bedeuten,
R₂
   Wasserstoff Hydroxy, Amino, Mercapto, einen geraden oder verzweigten, gesättigten oder ungesättigten aliphatischen Rest mit 1 - 15 Kohlenstoffatomen, der einoder mehrfach substituiert sein kann, eine Alkoxy-, Alkylamino-, Dialkylamino-, Alkylmercapto-, Alkylsulfinyl-, Alkylsulfonyl-, Alkenyl-, Alkinyl-, Alkenyloxy-, Alkenylmercapto-, Alkinyloxy-, Alkinylmercapto-, Alkylcarbonylamino-, Dialkylcarbonylamino-, Alkylaminocarbonyl-, Formyl-, Alkylcarbonyl-, Carboxyl-, Alkoxycarbonyl-, Alkenyloxycarbonyl-, Alkinyloxycarbonyl-, Carboxyalkyl-, Alkyloxycarbonylalkyl, Alkenyloxycarbonylalkyl-, Alkinyloxycarbonylalkyl-, Nitro-, Cyano-, Halogen-, Trifluormethyl-, oder Azidogruppe, Phenyl oder Phenylcarbonyl, das gegebenenfalls ein- oder mehrfach unabhängig voneinander substituiert sein kann, einen carbocyclischen mono-, bi- oder tricyclischen Rest mit 7-15 C-Atomen oder ein heterocyclisches mono-, bi- oder tricyclisches Ringsystem bedeutet,
R₄
   eine Gruppe CH₂CON(R₇)OXR₈ darstellt,
R₅ und R₆
   einzeln und unabhängig voneinander Wasserstoff, einen geraden oder verzweigten, gesättigten oder ungesättigten aliphatischen Rest mit 1 - 15 Kohlenstoffatomen, der ein- oder mehrfach substituiert sein kann, eine Alkoxy-, Alkylamino-, Dialkylamino-, Alkylmercapto-, Alkylsulfinyl-, Alkylsulfonyl-, Alkenyl-, Alkinyl-, Alkenyloxy-, Alkenylmercapto-, Alkinyloxy-, Alkinylmercapto-, Alkylcarbonylamino-, Dialkylcarbonylamino-, Alkylaminocarbonyl, Formyl-, Alkylcarbonyl-, Carboxyl-, Alkoxycarbonyl-, Alkenyloxycarbonyl-, Alkinyloxycarbonyl-, Carboxyalkyl-, Alkyloxycarbonylalkyl, Alkenyloxycarbonylalkyl-, Alkinyloxycarbonylalkyl-, Nitro-, Cyano-, Halogen-, Trifluormethyl-, oder Azidogruppe, Phenyl oder Phenylcarbonyl, das gegebenenfalls ein oder mehrfach unabhängig voneinander substituiert sein kann, einen carbocyclischen mono-, bi- oder tricyclischen Rest mit 7-15 C-Atomen oder ein heterocyclisches mono-, bi- oder tricyclisches Ringsystem,
R₇
   Wasserstoff oder einen Alkylrest,
X
   eine Valenzbindung, eine Alkylengruppe, eine Carbonylgruppe oder eine Alkylencarbonylgruppe,
R₈
   eine Alkylgruppe, die gewünschtenfalls ein- oder mehrfach gleich oder verschieden durch Hydroxy, Alkoxy, Amino, Alkylamino oder Dialkylamino substituiert sein kann, eine gewünschtenfalls in 2-Stellung mono- oder disubstituierte 1,3-Dioxolan-4-ylgruppe oder eine Gruppe PO(R₁₁)(R₁₂) oder PO(OR₁₃)(OR₁₄)
   sowie für den Fall, daß X nicht eine Valenzbindung darstellt, auch eine Hydroxy-, Alkoxy-, Hydroxyalkoxy, Alkanoyloxy-, Alkylcarbonylamino-, Alkoxycarbonyloxy-, Alkanoyloxyalkyl-, Carboxyvinylgruppe, eine gewünschtenfalls substituierte Aminoalkanoyloxy-, Aminoalkylphenyl- oder Aminoalkylbenzoyloxygruppe, eine Alkanoyloxyalkoxygruppe oder eine Gruppe NR₉R₁₀,
R₉ und R₁₀
   gleich oder verschieden Wasserstoff einen Alkylrest, der gewünschtenfalls ein- oder mehrfach gleich oder verschieden durch Hydroxy, Alkoxy, Amino, Alkylamino oder Dialkylamino substituiert sein kann,
   oder R₉ Wasserstoff und R₁₀ Hydroxy, Imidazolin-2-yl oder Tetrazol-5-yl bedeuten oder R₉ und R₁₀ gemeinsam mit dem Stickstoffatom, an das sie gebunden sind, einen heterocyclischen Ring bilden, der gewünschtenfalls durch Stickstoff oder Sauerstoff unterbrochen und durch Alkyl, Alkoxy, Hydroxy, Hydroxyalkyl, Oxo, Carboxy, Aminocarbonyl oder Alkoxycarbonyl substituiert und mit einem benzoiden Ring anelliert sein kann,
R₁₁ und R₁₂
   gleich oder verschieden sind und einen Alkylrest und
R₁₃ und R₁₄
   gleich oder verschieden sein können und Wasserstoff oder einen Alkylrest darstellen
   sowie deren Tautomere, Enantiomere, Diastereomere, Racemate und physiologisch verträgliche Salze oder Ester und Substanzen, die *in vivo* zu Verbindungen der Formel I hydrolysiert oder metabolisiert werden.

Die Erfindung betrifft auch Verfahren zur Herstellung der obigen Verbindungen, Arzneimittel, die solche Verbindungen enthalten sowie die Verwendung dieser Verbindungen bei der Herstellung von Arzneimitteln.

Überraschenderweise sind Verbindungen der allgemeinen Formel I oral wirksame Inhibitoren von Metalloproteasen.
Metalloproteasen spielen in vielen physiologischen und pathophysiologischen Prozessen eine große Rolle. Beispiele dafür sind das Angiotension Converting Enzyme (ACE) und die neutrale Endopeptidase (NEP, EC 3.4.24.11), die am Metabolismus einer Reihe von blutdruckregulierenden Peptiden (z. B. Angiotensin I und ANF (atrial natriuretic factor)) beteiligt sind. ACE katalysiert die Spaltung des Angiotensin I zu dem blutdrucksteigernden Angiotensin II. NEP ist für den Abbau des vasodilatierenden Peptids ANF verantwortlich. Das Endothelin Converting Enzyme (ECE) spaltet das endogene, inaktive big-Endothelin zu dem effektiven Vasokonstriktor Endothelin-1, einem aus 21 Aminosäuren bestehenden Peptid. Die Inhibierung dieser Enzyme hat eine große therapeutische Bedeutung zur Behandlung des Bluthochdrucks, der Herzinsuffizienz, des Nierenversagens und des Schlaganfalls. BMP-1 (bone morphogenic factor 1) wurde als Metalloprotease erkannt, die bei der Umwandlung von Procollagen in fibrilläres Collagen eine Rolle spielt. Inhibitoren dieses Enzyms sind für die Behandlung von Fibrosen und sklerotischen Prozessen geeignet und können auch die Narbenbildung bei der Wundheilung günstig beeinflussen. (*Proc. Natl. Acad. Sci. USA* **1996**, *93,* 5127, *Science* **199**6, *271*, 360).

Während Inhibitoren des ACE bereits therapeutisch angewandt werden (z. B. Captopril, Enalapril, (*Exp.Opin. Ther. Pat.* **1996**, *6, 1147*, sind für die Metalloproteasen wie NEP, ECE bisher keine klinisch verwendbaren Wirkstoffe bekannt, die frei von unerwünschten Nebenwirkungen und oral verfügbar sind. (Literaturübersichten: NEP: *Pharmacol. Rev.* **1993**, *45*, 87, ECE: *Bioorg. Med. Chem. Lett*. **1996**, *6*, 2317, zit. Lit. zu Inhibitoren vom Phosporamidontyp. Für das BMP-1 sind bisher noch keine niedermolekularen Inhibitoren bekannt.

Eine Gruppe der Metalloproteasen ist die der Matrixmetalloproteasen (MMPs). Verschiedene Gruppen von Metalloproteasen sind bekannt. Eine davon ist die der Matrixmetalloproteasen (MMPs). In normalem Gewebe besteht ein Gleichgewicht zwischen Auf- und Abbau der extrazellulären Matrix. Die extrazelluläre Matrix wird von wenigstens drei Gruppen Proteasen, nämlich den Collagenasen, Gelatinasen und den Stromelysinen abgebaut. Normalerweise sorgen spezifische Inhibitoren dieser Enzyme, wie z.B. α₂-Makroglobulin und TIMP (tissue inhibitor of metalloproteases) dafür, daß kein übermäßiger Abbau der extrazellulären Matrix stattfindet. Eine verwandte Gruppe von Proteasen ist die der Adamalysine mit ihrem prominentesten Mitglied, dem TNF-α converting enzyme (TACE) (Moss et al., *Nature* **1996**, *385*, 733).

Wenigstens 11 verschiedene und doch sehr homologe Matrixmetalloproteasen wurden charakterisiert, darunter die interstitial fibroblast collagenase (MMP-1, HFC), die neutrophile Collagenase (MMP-8, HNC), zwei Gelatinasen, Stromelysine (z.B. HSL-1) und Matrilysin (Birkedal-Hansen, H., Moore, W.G.I., Bodden, M.K. Windsor, L.J., Birkedahl-Hansen,B., DeCarlo, A., Engler, J.A., *Crit. Rev. Oral Biol. Med*. **1993**, *4*, 197-250). Diese Proteinasen teilen eine Reihe struktureller und funktionaler Eigenschaften, unterscheiden sich allerdings in ihrer Substratspezifität . Nur HNC und HFC spalten natives triple-helikales Collagen der Typen I, II und III. Dabei entstehen Fragmente von 3/4 und 1/4 der ursprünglichen Länge. Durch diesen Abbau wird der Schmelzpunkt des Collagens erniedrigt. Anschließend kann es durch andere matrixabbauende Enzyme angegriffen werden.

Der unkontrollierte exzessive Abbau dieser Matrizes ist für viele pathologische Erscheinungsbilder, wie z.B. rheumatoide Anthritis, Osteoarthritis, multiple Sklerose, Tumor Metastasierung, Cornea Ulzerationen, inflammatorische Prozesse und verschiedene Erkrankungen der Knochen und Zähne, typisch.

Die Pathogenese dieser Krankheiten sollte durch die Gabe von Metalloproteinaseinhibitoren positiv beeinflußt werden. In der Literatur finden sich inzwischen einige solcher Verbindungen (eine Übersicht findet sich z.B. bei Nigel R. A., Beeley et al., *Curr. Opin. Ther. Patents* **1994**, *4*(1), 7). Dabei handelt es sich vor allem um Peptide mit einem Hydroxamsäure-, Thiol- oder Phosphinrest als zinkbindende Gruppe (unter anderem z.B. WO-A-9209563 von Glycomed, EP-A-497192 von Hoffmann-LaRoche, WO-A-489577 von Celltech, EP-A-320118 von Beecham, US-A-4595700 von Searle).

Tumor Nekrose Faktor α (TNFα) ist ein proinflammatorisches Zytokin, das bei einer Vielzahl von Erkrankungen pathogenetische Bedeutung besitzt. Klinisch wurde in einer multizentrischen, randomisierten Doppel-Blind-Studie von Elliot et al. in *Lancet* **1994**, *344*, 1105-1110 gezeigt, daß ein neutralisierender Antikörper gegen TNFα eine schnelle und ausgeprägte Besserung der Krankheitssymptome bei Patienten mit rheumatoider Arthritis bewirken. Mittlerweile wurden von van Dullemen et al. in *Gastroenterolgy* **1995**, *109*, 129-135 klinische Daten publiziert, die eine therapeutische Wirkung solcher Antikörper bei Patienten mit Morbus Crohn belegen. Weiterhin wurde tierexperimentell gezeigt, daß Rolipram, das ebenfalls die Synthese von TNFα blockiert, in Tiermodellen für die Multiple Sklerose sehr gut wirksam ist. Thalidomid, eine weitere TNFα-hemmende Substanz, wird klinisch zur Behandlung von chronischer Graft versus Host-Erkrankung, bei der Behandlung von Lepra nodulare, und von Patienten mit Lupus erythematodes eingesetzt. Zudem wurde für diese Substanz gezeigt, daß sie die Proliferation von HIV unterdrückt.

Bei folgenden Krankheitsbildern scheint TNFα von direkter pathogenetischer Bedeutung zu sein:
Degenerative Gelenkserkrankungen, rheumatoide Arthritis, Entzündung, Allergie, ARDS, Asthma, Herzinfarkt, chronische Herzinsuffizienz, HIV-Infektion, Morbus Crohn, Ulzerative Colitis, Psoriasis, Dermatitis, Actinokeratose, Vaskulitiden, septischer Schock, Transplantatabstoßung, Multiple Sklerose, Ulzera, Diabetes, chronische Graft versus Host-Erkrankung, Lepra und andere Infektionskrankheiten, Lupus Erythematodes, Paradontose und bei anderen Erkrankungen.

Der klinische Einsatz von monoklonalen Anti-TNFα-Antikörpern kann nur parenteral erfolgen. Das Arzneimittel ist teuer herzustellen und erfordert eine aufwendige Vertriebslogistik (Kühlkette, Lagerung, Verfallzeit, etc.). Zudem wird bei 50 % der Patienten, die zwischen 2 und 4 Injektionen bekommen haben, das Auftreten neutralisierender HACAs (Human anti-chimeric antibodies) festgestellt. Dies führt dazu, daß die beschwerdefreien Phasen immer kürzer werden. Die Entwicklung von Rolipram als Anti-TNFα-Therapieprinzip ist durch dessen emetische Wirkung beeinträchtigt. Die teratogenen Nebenwirkungen von Thalidomid und die schwache TNFα-Blockade lassen eine klinische Entwicklung dieser Substanz ebenfalls schwierig erscheinen.

Überraschenderweise sind Verbindungen der allgemeinen Formel I oral verfügbare Inhibitoren von Metalloproteasen.

Die Verbindungen der allgemeinen Formel I, die Gegenstand der Erfindung sind, sind im folgenden näher beschrieben. Die Erfindung betrifft Substanzen der allgemeinen Formel I, 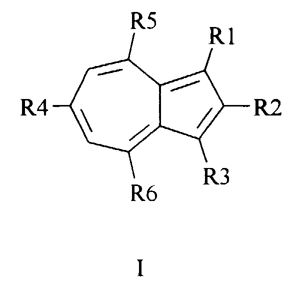 wobei
R₁ und R₃
   einzeln und unabhängig voneinander Wasserstoff Hydroxy, Amino, Mercapto, ein gerader oder verzweigter, gesättigter oder ungesättigter aliphatischer Rest mit 1 - 15 Kohlenstoffatomen, der ein- oder mehrfach durch Hydroxy, Amino, Mercapto, C₁-C₆-Alkoxy, C₁-C₆-Alkylamino, Di-C₁-C₆-alkylamino, C₁-C₆-Alkylmercapto, C₁-C₆-Alkylsulfinyl, C₁-C₆-Alkylsulfonyl, C₂-C₆-Alkenyl, C₂-C₆-Alkinyl, C₂-C₆-Alkenyloxy, C₂-C₆-Alkenylmercapto, C₂-C₆-Alkinyloxy, C₂-C₆-Alkinylmercapto, C₁-C₆-Alkylcarbonylamino, C₁-C₆-Alkylaminocarbonyl, Formyl, C₁-C₆-Alkylcarbonyl, Carboxyl, C₁-C₆-Alkoxycarbonyl, C₂-C₆-Alkenyloxycarbonyl, C₂-C₆-Alkinyloxycarbonyl, Carboxy-C₁-C₆-alkyl, C₁-C₆-Alkyloxycarbonyl-C₁-C₆-alkyl, C₂-C₆-Alkenyloxycarbonyl-C₁-C₆-alkyl, C₂-C₆-Alkinyloxycarbonyl-C₁-C₆-alkyl, Benzyloxy, Phenylmercapto, Phenyloxy, Nitro, Cyano, Halogen, Trifluormethyl, Azido, Formylamino oder Phenyl substituiert sein kann,
   eine C₁-C₆-Alkoxy-, C₁-C₆-Alkylamino-, Di-C₁-C₆-alkylamino-, C₁-C₆-Alkylmercapto-, C₁-C₆-Alkylsulfinyl-, C₁-C₆-Alkylsulfonyl-, C₂-C₆-Alkenyl-, C₂-C₆-Alkinyl-, C₂-C₆-Alkenyloxy-, C₂-C₆-Alkenylmercapto-, C₂-C₆-Alkinyloxy-, C₂-C₆-Alkinylmercapto-, C₁-C₆-Alkylcarbonylamino-, Di-C₁-C₆-alkylcarbonylamino-, C₁-C₆-Alkylaminocarbonyl-, Formyl-, C₁-C₆-Alkylcarbonyl-, Carboxyl-, C₁-C₆-Alkoxycarbonyl-, C₂-C₆-Alkenyloxycarbonyl-, C₂-C₆-Alkinyloxycarbonyl-, Carboxy-C₁-C₆-alkyl-, C₁-C₆-Alkyloxycarbonyl-C₁-C₆-alkyl-, C₂-C₆-Alkenyloxycarbonyl-C₁-C₆-alkyl-, C₂-C₀-Alkinyloxycarbonyl-C₁-C₆-alkyl-, Nitro-, Cyano-, Halogen-, Trifluormethyl- oder Azidogruppe,
   Phenyl oder Phenylcarbonyl, das gegebenenfalls ein- oder mehrfach unabhängig voneinander durch Hydroxy, Amino, Mercapto, C₁-C₆-Alkoxy, C₁-C₆-Alkylamino, Di-C₁-C₆-alkylamino, C₁-C₆-Alkylmercapto, C₁-C₆-Alkylsulfinyl, C₁-C₆-Alkylsulfonyl, C₂-C₆-Alkenyl, C₂-C₆-Alkinyl, C₂-C₆-Alkenyloxy, C₂-C₆-Alkenylmercapto, C₂-C₆-Alkinyloxy, C₂-C₆-Alkinylmercapto, C₁-C₆-Alkylcarbonylamino, C₁-C₀-Alkylaminocarbonyl, Formyl, C₁-C₆-Alkylcarbonyl, Carboxyl, C₁-C₆-Alkoxycarbonyl-, C₂-C₆-Alkenyloxycarbonyl, C₂-C₆-Alkinyloxycarbonyl-, Carboxy-C₁-C₆-alkyl, C₁-C₆-Alkyloxycarbonyl-C₁-C₆-alkyl, C₂-C₆-Alkenyloxycarbonyl-C₁-C₆-alkyl, C₂-C₆-Alkinyloxycarbonyl-C₁-C₆-alkyl, Benzyloxy, Phenylmercapto, Phenyloxy, Nitro, Cyano, Halogen, Trifluormethyl, Azido, Formylamino, Carboxy oder Phenyl substituiert sein kann,
   einen carbocyclischen mono-, bi- oder tricyclischen Rest mit 7-15 C-Atomen oder ein heterocyclisches mono-, bi- oder tricyclisches Ringsystem darstellen kann, wobei die ungesättigten bzw. aromatischen Carbo- und Heterocyclen partiell oder vollstandig hydriert sein können,
R₂
   Wasserstoff, Hydroxy, Amino, Mercapto, einen geraden oder verzweigten, gesättigten oder ungesättigten aliphatischen Rest mit 1 - 15 Kohlenstoffatomen, der ein- oder mehrfach durch Hydroxy, Amino, Mercapto, C₁-C₆-Alkoxy, C₁-C₆-Alkylamino, Di-C₁-C₆-alkylamino, C₁-C₆-Alkylmercapto, C₁-C₆-Alkylsulfinyl, C₁-C₆-Alkylsullönyl, C₂-C₆-Alkenyl, C₂-C₆-Alkinyl, C₂-C₆-Alkenyloxy, C₂-C₆-Alkenylmercapto, C₂-C₆-Alkinyloxy, C₂-C₆-Alkinylmercapto, C₁-C₆-Alkylcarbonylamino, C₁-C₆-Alkylaminocarbonyl, Formyl, C₁-C₆-Alkylcarbonyl, Carboxyl, C₁-C₆-Alkoxycarbonyl-, C₂-C₆-Alkenyloxycarbonyl, C₂-C₆-Alkinyloxycarbonyl, Carboxy-C₁-C₆-alkyl, C₁-C₆-Alkyloxycarbonyl-C₁-C₆-alkyl, C₂-C₆-Alkenyloxycarbonyl-C₁-C₆-alkyl, C₂-C₆-Alkinyloxycarbonyl-C₁-C₆-alkyl, Benzyloxy, Phenylmercapto, Phenyloxy, Nitro, Cyano, Halogen, Trifluormethyl, Azido, Formylamino oder Phenyl substituiert sein kann,
   eine C₁-C₆-Alkoxy-, C₁-C₆-Alkylamino-, Di-C₁-C₆-alkylamino-, C₁-C₆-Alkylmercapto-, C₁-C₆-Alkylsulfinyl-, C₁-C₆-Alkylsulfonyl-, C₂-C₆-Alkenyl-, C₂-C₆-Alkinyl-, C₂-C₆-Alkenyloxy-, C₂-C₆-Alkenylmercapto-, C₂-C₆-Alkinyloxy-, C₂-C₆-Alkinylmercapto-, C₁-C₆-Alkylcarbonylamino-, Di-C₁-C₆-alkylcarbonylamino-, C₁-C₆-Alkylaminocarbonyl-, Formyl-, C₁-C₆-Alkylcarbonyl-, Carboxyl-, C₁-C₆-Alkoxycarbonyl-, C₂-C₆-Alkenyloxycarbonyl-, C₂-C₆-Alkinyloxycarbonyl-, Carboxy-C₁-C₆-alkyl-, C₁-C₆-Alkyloxycarbonyl-C₁-C₆-alkyl-, C₂-C₆-Alkenyloxycarbonyl-C₁-C₆-alkyl-, C₂-C₆-Alkinyloxycarbonyl-C₁-C₆-alkyl-, Nitro-, Cyano-, Halogen-, Trifluormethyl- oder Azidogruppe,
   Phenyl oder Phenylcarbonyl, das gegebenenfalls ein- oder mehrfach unabhängig voneinander durch Hydroxy, Amino, Mercapto, C₁-C₆-Alkoxy, C₁-C₆-Alkylamino, Di-C₁-C₆-alkylamino, C₁-C₆-Alkylmercapto, C₁-C₆-Alkylsulfinyl, C₁-C₆-Alkylsulfonyl, C₂-C₆-Alkenyl, C₂-C₆-Alkinyl, C₂-C₆-Alkenyloxy, C₂-C₆-Alkenylmercapto, C₂-C₆-Alkinyloxy, C₂-C₆-Alkinylmercapto, C₁-C₆-Alkylcarbonylamino, C₁-C₆-Alkylaminocarbonyl, Formyl, C₁-C₆-Alkylcarbonyl, Carboxyl, C₁-C₆-Alkoxycarbonyl, C₂-C₆-Alkenyloxycarbonyl, C₂-C₆-Alkinyloxycarbonyl, CarboxyC₁-C₆-alkyl, C₁-C₆-Alkyloxycarbonyl-C₁-C₆-alkyl, C₂-C₆-Alkenyloxycarbonyl-C₁-C₆-alkyl, C₂-C₆-Alkinyloxycarbonyl-C₁-C₆-alkyl, Benzyloxy, Phenylmercapto, Phenyloxy, Nitro, Cyano, Halogen, Trifluormethyl, Azido, Formylamino, Carboxy oder Phenyl substituiert sein kann.
   einen carbocyclischen mono-, bi- oder tricyclischen Rest mit 7-15 C-Atomen oder ein heterocyclisches mono-, bi- oder tricyclisches Ringsystem darstellen kann, wobei die
   ungesättigten bzw. aromatischen Carbo- und Heterocyclen partiell oder vollständig hydriert sein können.
R₄
   bedeutet eine Gruppe CH₂CON(R₇)OXR₈,
R₅ und R₆
   bedeuten einzeln und unabhängig voneinander Wasserstoff einen geraden oder verzweigten, gesättigten oder ungesättigten aliphatischen Rest mit 1 - 15 Kohlenstoffatomen, der ein oder mehrfach durch Hydroxy, Aniino, Mercapto, C₁-C₆-Alkoxy, C₁-C₆-Alkylamino, Di-C₁-C₆-alkylamino, C₁-C₆-Alkylmercapto, C₁-C₆-Alkylsulfinyl, C₁-C₆-Alkylsulfonyl, C₂-C₆-Alkenyl, C₂-C₆-Alkinyl, C₂-C₆-Alkenyloxy, C₂-C₆-Alkenylmercapto, C₂-C₆-Alkinyloxy, C₂-C₆-Alkinylmercapto, C₁-C₆-Alkylcarbonylamino, C₁-C₆-Alkylaminocarbonyl, Formyl, C₁-C₆-Alkylcarbonyl, Carboxyl, C₁-C₆-Alkoxycarbonyl, C₂-C₆-Alkenyloxycarbonyl-, C₂-C₆-Alkinyloxycarbonyl-, Carboxy-C₁-C₆-alkyl, C₁-C₆-Alkyloxycarbonyl-C₁-C₆-alkyl, C₂-C₆-Alkenyloxycarbonyl-C₁-C₆-alkyl, C₂-C₆-Alkinyloxycarbonyl-C₁-C₆-alkyl, Benzyloxy, Phenylmercapto, Phenyloxy, Nitro, Cyano, Halogen, Trifluormethyl, Azido, Formylamino oder Phenyl substituiert sein kann,
   eine C₁-C₆-Alkoxy-, C₁-C₆-Alkylamino-, Di-C₁-C₆-alkylamino-, C₁-C₆-Alkylmercapto-, C₁-C₆-Alkylsulfinyl-, C₁-C₆-Alkylsulfonyl-, C₂-C₆-Alkenyl-, C₂-C₆-Alkinyl-, C₂-C₆-Alkenyloxy-, C₂-C₆-Alkenylmercapto-, C₂-C₆-Alkinyloxy-, C₂-C₆-Alkinylmercapto-, C₁-C₆-Alkylcarbonylamino-, Di-C₁-C₆-alkylcarbonylamino-, C₁-C₆-Alkylaminocarbonyl-, Formyl-, C₁-C₆-Alkylcarbonyl-, Carboxyl-, C₁-C₆-Alkoxycarbonyl-, C₂-C₆-Alkenyloxycarbonyl-, C₂-C₆-Alkinyloxycarbonyl-, Carboxy-C₁-C₆-alkyl-, C₁-C₆-Alkyloxycarbonyl-C₁-C₆-alkyl-, C₂-C₆-Alkenyloxycarbonyl-C₁-C₆-alkyl-, C₂-C₆-Alkinyloxycarbonyl-C₁-C₆-alkylgruppe,
   Phenyl, das gegebenenfalls ein- oder mehrfach unabhängig voneinander durch Hydroxy, Amino, Mercapto, C₁-C₆-Alkoxy, C₁-C₆-Alkylamino, Di-C₁-C₆-alkylamino, C₁-C₆-Alkylmercapto, C₁-C₆-Alkylsulfinyl, C₁-C₆-Alkylsulfonyl, C₂-C₆-Alkenyl, C₂-C₆-Alkinyl, C₂-C₆-Alkenyloxy, C₂-C₆-Alkenylmercapto, C₂-C₆-Alkinyloxy, C₂-C₆-Alkinylmercapto, C₁-C₆-Alkylcarbonylamino, C₁-C₆-Alkylaminocarbonyl, Formyl, C₁-C₆-Alkylcarbonyl, Carboxyl, C₁-C₆-Alkoxycarbonyl, C₂-C₆-Alkenyloxycarbonyl, C₂-C₆-Alkinyloxycarbonyl, Carboxy-C₁-C₆-alkyl, C₁-C₆-Alkyloxycarbonyl-C₁-C₆-alkyl, C₂-C₆-Alkenyloxycarbonyl- C₁-C₆-alkyl, C₂-C₆-Alkinyloxycarbonyl-C₁-C₆-alkyl, Benzyloxy, Phenylmercapto, Phenyloxy, Nitro, Cyano, Halogen, Trifluormethyl, Azido, Formylamino, Carboxy oder Phenyl substituiert sein kann,
   einen carbocyclischen mono-, bi- oder tricyclischen Rest mit 7-15 C-Atomen oder ein heterocyclisches mono-, bi- oder tricyclisches Ringsystem darstellen kann, wobei die ungesättigten bzw. aromatischen Carbo- und Heterocyclen partiell oder vollständig hydriert sein können.
R₇
   symbolisiert Wasserstoff oder einen C₁-C₆-Alkylrest.
X
   steht für eine Valenzbindung, eine C₁-C₆-Alkylengruppe, eine Carbonylgruppe oder eine C₁-C₆-Alkylencarbonylgruppe.
R₈
   kann einen C₁-C₆-Alkylrest, der gewünschtenfalls ein- oder mehrfach gleich oder verschieden durch Hydroxy, C₁-C₆-Alkoxy, Amino, C₁-C₆-Alkylamino oder Di-C₁-C₆-alkylamino substituiert sein kann, eine gewünschtenfalls in 2-Stellung mono- oder disubstituierte 1,3-Dioxolan-4-ylgruppe oder eine Gruppe PO(R₁₁)(R₁₂) oder PO(OR₁₃)(OR₁₄)
   sowie für den Fall, daß X nicht eine Valenzbindung bedeutet, auch eine Hydroxy-, C₁-C₆-Alkoxy-, Hydroxy-C₁-C₆-alkoxy-, C₁-C₆-Alkanoyloxy-, C₁-C₆-Alkylcarbonylamino-, C₁-C₆-Alkoxycarbonyloxy-, C₁-C₆-Alkanoyloxy-C₁-C₆-alkyl- oder Carboxyvinylgruppe, eine gewünschtenfalls N-substituierte Amino-C₁-C₆-alkanoyloxy-, Amino-C₁-C₆-alkylphenyl- oder Amino-C₁-C₆-alkyl-benzoylgruppe, eine C₁-C₆-Alkanoyloxy-C₁-C₆-alkoxygruppe oder eine Gruppe NR₉R₁₀ bedeuten.
R₉ und R₁₀
   können gleich oder verschieden sein und für Wasserstoff, einen C₁-C₆-Alkylrest, der gewünschtenfalls ein- oder mehrfach gleich oder verschieden durch Hydroxy, C₁-C₆-Alkoxy, Amino, C₁-C₆-Alkylamino oder Di-C₁-C₆-alkylamino substituiert sein kann, stehen oder R₉ bedeutet Wasserstoff und R₁₀ Hydroxy, Imidazolin-2-yl oder Tetrazol-5-yl oder R₉ und R₁₀ bilden gemeinsam mit dem Stickstoffatom, an das sie gebunden sind, einen heterocyclischen Ring, der gewünschtenfalls durch Stickstoff oder Sauerstoff unterbrochen und durch C₁-C₆-Alkyl, C₁-C₆-Alkoxy, Hydroxy, Hydroxy-C₁-C₆-alkyl, Oxo, Carboxy, Aminocarbonyl oder C₁-C₆-Alkoxycarbonyl substituiert und mit einem benzoiden Ring anelliert sein kann.
R₁₁ und R₁₂
   sind gleich oder verschieden und stehen für einen C₁-C₆-Alkylrest.
R₁₃ und R₁₄
   sind gleich oder verschieden und bedeuten Wasserstoff oder einen C₁-C₆-Alkylrest.

Ein aliphatischer Rest bedeutet einen geradkettigen, verzweigten oder cyclischen Alkyl-, Alkenyl- oder Alkinylrest mit 1-15, vorzugsweise 1-10 Kohlenstoffatomen, wie z. B. der Methyl-, Ethyl-, Propyl-, Isopropyl-, Butyl-, *s*ec-Butyl-, *tert*-Butyl-, Pentyl-, Hexyl-, Heptyl-, Octyl-, Nonyl-, Decyl-, Cyclopropyl-, Cyclobutyl-, Cyclopentyl- und Cyclohexylrest. Ungesättigte Reste sind z. B. der Vinyl-, Allyl-, Dimethylallyl-, Butenyl-, Isobutenyl-, Pentenyl-, Ethinyl- oder Propinylrest.

C₁-C₆-Alkylreste in C₁-C₆-Alkyl, C₁-C₆-Alkoxy, C₁-C₆-Alkylamino, Di-C₁-C₆-alkylamino, C₁-C₆-Alkylmercapto, C₁-C₆-Alkylsulflnyl, C₁-C₆-Alkylsulfonyl, C₁-C₆-Alkylcarbonylamino, C₁-C₆-Alkylaminocarbonyl, C₁-C₆-Alkoxycarbonyl, Carboxy-C₁-C₆-alkyl, C₁-C₆-Alkyloxycarbonyl-C₁-C₆-alkyl, C₂-C₆-Alkenyloxycarbonyl-C₁-C₆-alkyl, C₂-C₆-Alkinyloxycarbonyl-C₁-C₆-alkyl, C₁-C₆-Alkanoyloxy, C₁-C₆-Alkoxycarbonyloxy, C₁-C₆-Alkoxy-C₁-C₆-alkyl, C₁-C₆-Alkanoyloxy-C₁-C₆-alkyl, Amino-C₁-C₆-alkanoyloxy, Amino-C₁-C₆-alkylphenyl, Amino-C₁-C₆-alkylbenzoyl, C₁-C₆-Alkanoyloxy-C₁-C₆-alkoxy, Hydroxy-C₁-C₆-alkyl bedeuten geradkettige, verzweigte oder cyclische Reste. Bevorzugt sind die Methyl-, Ethyl-, Propyl-, Isopropyl-, Butyl-, *sec*- oder *tert*-Butyl-, Pentyl-, Hexyl-, Cyclopropyl-, Cyclobutyl-, Cyclopentyl-, Cyclohexyl-, Methoxy-, Ethoxy-, Propyloxy-, Isopropyloxy-, Butyloxy-, *sec*-Butyloxy-, *tert*-Butyloxy-, Methoxycarbonyl-, Ethoxycarbonyl-, Propyloxycarbonyl-, Butyloxycarbonyl-, Carboxymethyl-, Carboxyethyl-, Carboxypropyl-, Methoxycarbonylethyl-, Ethoxycarbonylethyl-, Methoxycarbonylpropyl-, Ethoxycarbonylpropyl-, Carboxymethoxy-, Carboxyethoxy-, Carboxypropyloxy-, Methoxycarbonylmethoxy-, Ethoxycarbonylethoxy-, Propoxycarbonylmethoxy-, Methoxycarbonylethoxy-, Ethoxycarbonylethoxy-, Aminomethyl-, Aminoethyl-, Aminopropyl-, Methylmercapto-, Ethylmercapto-, Propylmercaptogruppe.

C₂-C₆-Alkenyl- und C₂-C₆-Alkinylreste in C₂-C₆-Alkenyl, C₂-C₆-Alkinyl, C₂-C₆-Alkenyloxy, C₂-C₆-Alkenylmercapto, C₂-C₆-Alkinyloxy, C₂-C₆-Alkinylmercapto, C₂-C₆-Alkenyloxycarbonyl, C₂-C₆-Alkinyloxycarbonyl, C₂-C₆-Alkenyloxycarbonyl-C₁-C₆-alkyl-, C₂-C₆-Alkinyloxycarbonyl-C₁-C₆-alkyl bedeuten geradkettige, verzweigte oder cyclische Reste Bevorzugt sind die Vinyl-, Propenyl-, Butenyl-, Pentenyl-, Hexenyl-, Ethinyl-, Propargyl-, Vinyloxy-, Allyloxy-, Propargyloxy-, Allyloxycarbonyl-, Propargyloxycarbonyl-, Allyloxycarbonylmethyl-, Allyloxycarbonylethyl-, Allyloxycarbonylpropyl-, Propargyloxycarbonylmethyl-, Propargyloxycarbonylethyl- und Propargyloxycarbonylpropylgruppe.

Ein carbocyclischer Ring mit 7-15 C-Atomen kann mono-, bi- oder tricyclisch sein und pro Ring jeweils 5-7 C-Atome aufweisen. Dieser Ring kann aromatisch oder ganz oder teilweise gesättigt sein. Bevorzugt sind der Naphthyl-, Anthracenyl-, Phenanthrenyl-, Fluorenyl-, Indenyl-, Acenaphtylenyl-, Norbornyl-, Adamantylring oder eine C₃-C₇-Cycloalkyl oder C₅-C₈-Cycloalkenylgruppe. Der carbocyclische Ring kann darüberhinaus 1-3 fach substituiert sein, wobei die Substituenten unabhängig voneinander ein gerader oder verzweigter, gesättigter oder ungesättigter aliphatischer Rest mit 1-9 Kohlenstoffatomen, eine Hydroxy-, Amino-, Mercapto-, C₁-C₆-Alkoxy-, C₁-C₆-Alkylamino-, Di-C₁-C₆-alkylamino-, C₁-C₆-Alkylmercapto-, C₁-C₆-Alkylsulfinyl-, C₁-C₆-Alkylsulfonyl-, C₂-C₆-Alkenyl-, C₂-C₆-Alkinyl-, C₂-C₆-Alkenyloxy-, C₂-C₆-Alkenylmercapto-, C₂-C₆-Alkinyloxy-, C₂-C₆-Alkinylmercapto-C₁-C₆-Alkylcarbonylamino-, C₁-C₆-Alkylaminocarbonyl-, Carbonyl-, C₁-C₆-Alkylcarbonyl-, Carboxyl-, C₁-C₆-Alkoxycarbonyl-, C₂-C₆-Alkenyloxycarbonyl-, C₂-C₆-Alkinyloxycarbonyl-, Carboxy-C₁-C₆-alkyl-, C₁-C₆-Alkyloxycarbonyl-C₁-C₆-alkyl-, C₂-C₆-Alkenyloxycarbonyl-C₁-C₆-alkyl-, C₂-C₆-Alkinyloxycarbonyl-C₁-C₆-alkyl-, Benzyloxy-, Phenylmercapto-, Phenyloxy-, Nitro-, Cyano-, Halogen-, Trifluormethyl-, Azido-, Formylamino-, oder Phenylgruppe sein können. Alkylenreste in C₁-C₆-Alkylen- und C₁-C₆-Alkylencarbonylgruppen sind beispielsweise die Methylen-, 1,1-Ethylen-, 1,2-Ethylen-, 1,1-Propylen-, 1,2-Propylen-, 1,3-Propylen-, 1,1-Butylen-, 1,2-Butylen-, 1,3-Butylen-, 1,4-Butylen-, 1,1-Pentylen- und 1,1-Hexylengruppe.

Unter einem heterocyclischen mono-, bi- oder tricyclischen Ringsystem versteht man ein gesättigtes oder ungesättigtes Ringsystem mit fünf- bis siebengliedrigen Ringen, welches 1-4 gleiche oder verschiedene Heteroatome wie Stickstoff, Sauerstoff oder Schwefel enthält, wie z.B. das Pyridin-, Pyrimidin-, Pyridazin-, Pyrazin-, Triazin-, Pyrrol-, Pyrazol-, Piperidin, Morpholin lmidazol-, Triazol-, Thiazol-, Oxazol-, Isoxazol-, Oxadiazol-, Furazan-, Furan-, Thiophen-, Indol-, Chinolin-, Isochinolin-, Cumaron-, Thionaphten-, Benzoxazol-, Benzthiazol-, Indazol-, Benzimidazol-, Benztriazol-, Chromen-, Phthalazin-, Chinazolin-, Chinoxalin-, Methylendioxybenzol-, Carbazol-, Acridin-, Phenoxazin-, Phenothiazin-, Phenazin-, oder Purinsystem, wobei die ungesättigten bzw. aromatischen Carbo- und Heterocyclen partiell oder vollständig hydriert sein können. Das heterocyclische System kann darüberhinaus ein oder mehrfach substituiert sein, wobei die Substituenten unabhängig voneinander ein gerader oder verzweigter, gesättigter oder ungesättigter aliphatischer Rest mit 1-9 Kohlenstoffatomen, eine Hydroxy-, Amino-, Mercapto-, C₁-C₆-Alkoxy-, C₁-C₆-Alkylamino-, Di-C₁-C₆-alkylamino-, C₁-C₆-Alkylmercapto-, C₁-C₆-Alkylsulfinyl-, C₁-C₆-Alkylsulfonyl, C₂-C₆-Alkenyl-, C₂-C₆-Alkinyl-, C₂-C₆-Alkenyloxy-, C₂-C₆-Alkenylmercapto-, C₂-C₆-Alkinyloxy-, C₂-C₆-Alkinylmercapto-, C₁-C₆-Alkylcarbonylamino-, C₁-C₆-Alkylaminocarbonyl-, Formyl-, C₁-C₆-Alkylcarbonyl-, Carboxyl-, C₁-C₆-Alkoxycarbonyl-, C₂-C₆-Alkenyloxycarbonyl-, C₂-C₆-Alkinyloxycarbonyl-, Carboxy-C₁-C₆-alkyl-, C₁-C₆-Alkyloxycarbonyl-C₁-C₆-alkyl-, C₂-C₆-Alkenyloxycarbonyl-C₁-C₆-alkyl-, C₂-C₆-Alkinyloxycarbonyl-C₁-C₆-alkyl-, Benzyloxy-, Phenylmercapto-, Phenyloxy-, Nitro-, Cyano-, Halogen-, Trifluormethyl-, Azido-, Formylamino-, oder Phenylgruppe sein können. Bevorzugt sind Pyrrolidin, Piperidin, Piperazin, Morpholin, Hexahydroazepin, Tetrahydrofuran, Tetrahydropyran, Tetrahydrothiophen, 4,5-Dihydroimidazol, Pyrrol, Imidazol, Pyrazin, Pyrimidin, Pyridazin, 1*H*-Azepin, 3*H*-Azepin, 1,2-Diazepin, 1,4-Diazepin, Furan, Thiophen. Oxazol, Isoxazol, Thiazol, Isothiazol, Pyrazol, Pyrrolidinon, Imidazolidinon, Piperidinon, Indol, Purin, Chinolin und Isochinolin.

Bevorzugt sind Verbindungen der allgemeinen Formel I, in denen R₁ und/oder R₃ Wasserstoff oder einen C₁-C₆-Alkoxycarbonylrest bedeutet, R₂ Wasserstoff, Amino, C₁-C₆-Alkylcarbonylamino, Di-C₁-C₆-alkylcarbonylamino bedeutet, R₅ und R₆ Wasserstoff oder C₁-C₆-Alkoxy, R₇ Wasserstoff oder C₁-C₆-Alkyl, X eine Valenzbindung, eine C₁-C₆-Alkylengruppe, eine Carbonylgruppe oder eine C₁-C₆-Alkylencarbonylgruppe, R₈ einen C₁-C₆-Alkylrest, der ein- oder mehrfach gleich oder verschieden durch Hydroxy, C₁-C₆-Alkoxy, Amino, C₁-C₆-Alkylamino oder Di-C₁-C₆-alkylamino substituiert sein kann, Hydroxy, C₁-C₆-Alkoxy, Hydroxy-C₁-C₆-alkoxy, eine 2,2-Dialkyl-1,3-dioxolan-4-ylgruppe oder eine Gruppe PO(OR₃)(OR₄) oder NR₉R₁₀ bedeuten, wobei

R₉ und R₁₀ für Wasserstoff einen C₁-C₆-Alkylrest, der gewünschtenfalls ein- oder mehrfach gleich oder verschieden durch Hydroxy oder C₁-C₆-Alkoxy substituiert sein kann, stehen oder R₉ und R₁₀ gemeinsam mit dem Stickstoffatom, an das sie gebunden sind, einen heterocyclischen Ring bilden, der gewunschtenfalls durch Stickstoff oder Sauerstoff unterbrochen und durch C₁-C₆-Alkyl, C₁-C₆-AIkoxy, Hydroxy, Hydroxy-C₁-C₆-alkyl, Oxo, Carboxy, Aminocarbonyl oder C₁-C₆-Alkoxycarbonyl substituiert und mit einem benzoiden Ring anelliert sein kann und
R₁₃ und R₁₄ gleich oder verschieden sind und Wasserstoff oder einen C₁-C₆-Alkylrest bedeuten.

Insbesonders bevorzugte Substituenten für R₁ und/oder R₃ sind Carbonsäuremethyl-, Carbonsäureethyl- sowie Carbonsäure*t*butylester, für R₂ Amino, R₅ und R₆ Wasserstoff, R₇ Wasserstoff oder Methyl, X Methylcarbonyl, Ethyl oder eine Valenzbindung, R₈ Hydroxy, Ethoxy, 2,3-Dihydoxypropyl, 2-Hydroxy-3-methoxypropyl, 2,2-Dimethyl-1,3-dioxolan-4-yl, 3-Dimethylamino-2-hydroxypropyl und R₉ und R₁₀ bilden bevorzugt gemeinsam einen Pyrrolidin-, Piperidin- oder Morpholinring oder eine Phthalimidogruppe oder bedeuten bevorzugt Methoxyethyl, Methyl oder Hydroxyethyl.

Unter den physiologisch verträglichen Salzen der allgemeinen Formel I versteht man beispielsweise Formiate, Acetate, Caproate, Oleate, Lactate oder Salze von Carbonsäuren mit bis zu 18 Kohlenstoffatomen oder Salze von Dicarbonsäuren und Tricarbonsäuren wie Citrate, Malonate und Tartrate oder Alkansulfonate mit bis zu 10 Kohlenstoffatomen oder p-Toluolsulfonate oder Salicylate oder Trifluoracetate oder Salze von physiologisch verträglichen Mineralsäuren wie Salzsäure, Bromwasserstoffsäure, Iodwasserstoffsäure, Schwefelsäure, Phosphorsäure. Die Verbindungen der Formel I mit freier Carboxylgruppe können auch Salze mit physiologisch verträglichen Basen bilden. Beispiele solcher Salze sind Alkalimetall-, Erdalkalimetall-, Ammonium- und Alkylammoniumsalze, wie das Natrium-, Kalium-, Calcium- oder Tetramethylammoniumsalz.

Zu reinen Enantiomern der Verbindungen der Formel I kommt man entweder durch Racematspaltung (über Salzbildung mit optisch aktiven Säuren oder Basen) oder indem man in die Synthese optisch aktive Ausgangsstoffe einsetzt.

Die Verbindungen der Formel I können solvatisiert, insbesondere hydratisiert sein. Die Hydratisierung kann im Zuge der Herstellung erfolgen oder allmählich als Folge hygroskopischer Eigenschaften einer zunächst wasserfreien Verbindung der Formel I auftreten.

Verbindungen der Formel I können in flüssiger, fester oder in Form von Aerosolen oral, enteral, parenteral, topisch, nasal, pulmonal oder rectal in allen üblichen nichttoxischen pharmazeutisch akzeptierten Trägermaterialien, Adjuvantien und Zusätzen verabreicht werden. Der Begriff parenteral umfaßt dabei subcutane , intravenöse und intramuskuläre Zuführ oder Infusionen. Orale Applikationsformen sind z.B. in W.A. Ritschel, Die Tablette, 1966, Aulendorf, beschrieben und können z.B. Tabletten, Kapseln, Dragees, Sirupe, Lösungen, Suspensionen, Emulsionen, Elixiere etc. sein, die einen oder mehrere Zusätze aus den folgenden Gruppen enthalten können, wie z.B. Geschmacksstoffe, Süßstoffe, Farbstoffe und Konservierungsmittel Orale Applikationsformen enthalten den wirksamen Bestandteil zusammen mit nichttoxischen, pharmazeutisch akzeptierten Trägermaterialien, die zur Herstellung von Tabletten, Kapseln, Dragees usw. geeignet sind, wie z.B. Calciumcarbonat, Natriumcarbonat, Lactose, Calciumphosphat oder Natriumphosphat; Stärke, Mannit, Methylcellulose, Talkum, hochdisperse Kieselsäuren, höhermolekulare Fettsäuren (wie Stearinsäure), Erdnußöl, Olivenöl, Paraffin, Miglyol, Gelatine, Agar-Agar, Magnesiumstearat, Bienenwachs, Cetylalkohol, Lecithin, Glycerin, tierische und pflanzliche Fette, feste hochmolekulare Polymere (wie Polyethylenglykole). Tabletten, Kapseln, Dragees usw. können mit einem entsprechenden Überzug, wie z. B. Glycerylmonostearat oder Glyceryldistearat versehen werden, so daß unerwünschte Nebenwirkungen im Magen verhindert werden, oder es durch die verzögerte Absorption im Gastrointestinaltrakt zu einer längeren Wirkungsdauer kommt. Als Injektionsmedium kommen vorzugsweise sterile injizierbare wässrige oder ölige Lösungen oder Suspensionen zur Anwendung, welche die üblichen Zusätze, wie Stabilisierungsmittel und Lösungsvermittler enthalten. Derartige Zusätze können z.B. Wasser, isotonische Kochsalzlösung, 1,3-Butandiol, Fettsäuren (wie Ölsäure), Mono- und Diglyceride, oder Miglyol sein. Für die rectale Anwendung können alle geeigneten nicht irritierenden Zusätze verwendet werden, die bei normalen Temperaturen fest und bei Rectaltemperatur flüssig sind, wie z. B. Kakaobutter und Polyethylenglykol. Für die Anwendung als Aerosol kommen die pharmazeutisch üblichen Trägermedien zur Anwendung. Für den äußerlichen Gebrauch finden Cremes, Tinkturen, Gele, Lösungen oder Suspensionen usw. mit den pharmazeutisch üblichen Zusätzen Anwendung.

Die Dosierung kann von verschiedenen Faktoren, wie Applikationsweise, Spezies, Alter und/oder individuellem Zustand abhängen. Die täglich zu verabreichende Dosis wirksamer Substanz liegt bei 0.01 mg bis ungefähr 100 mg/kg Körpergewicht, vorzugsweise bei 0.1 bis 10 mg/kg Körpergewicht und kann auf einmal oder mehrere Male verteilt appliziert werden.

Die Herstellung der Verbindungen der Formel I erfolgt nach an sich bekannten Methoden, wie sie in der Literatur (z.B. in Standardwerken wie Houben-Weyl, Methoden der Organischen Chemie, Georg Thieme Verlag, Stuttgart; Organic Reactions, John Wiley & Sons, Inc., New York) beschrieben sind, und zwar unter Reaktionsbedingungen, wie sie für die genannten Umsetzungen bekannt und geeignet sind und ist auch in EP 97110533.3 beschrieben. Dabei kann man auch von an sich bekannten, hier nicht näher erwähnten, Varianten Gebrauch machen. Weiterhin kann eine Verbindung der Formel I nach an sich bekannten Methoden in eine andere Verbindung der Formel I umgewandelt werden.

Das erfindungsgemäße Verfahren zur Herstellung von Verbindungen der Formel I ist dadurch gekennzeichnet, daß man in an sich bekannter Weise eine Verbindung der Formel I, in der R₁, R₂, R₃, R₅ und R₆ die angegebene Bedeutung haben und R₄ eine Gruppe CH₂CON(R₇)OH darstellt, worin R₇ die angegebene Bedeutung hat, oder ein Alkali- oder Erdalkalimetallsalz hiervon,
mit einer Verbindung Y-X-R₈, worin X und R₈ die angegebene Bedeutung haben und Y einen reaktiven Rest darstellt, umsetzt
und anschließend gewünschtenfalls die erhaltene Verbindung der Formel I in eine andere, durch den Anspruch definierte Verbindung der Formel I umwandelt sowie gegebenenfalls in ein pharmakologisch verträgliches Salz überführt.

Als reaktive Reste Y kommen alle Reste infrage, die in Alkylierungs- oder Acylierungsreaktionen als Austrittsgruppen Verwendung finden, wie beispielsweise Halogenatome, die Azidogruppe, Alkoxy-, Aryloxy-, Alkylthio-, Arylthio-, Acyloxygruppen, der Imidazolrest, die Mesyloxy-, Tosyloxy- oder Brosyloxygruppe und anorganische Säurereste wie beispielsweise Sulfate und Phosphate.

Die Umsetzung erfolgt zweckmäßig in einem Lösungsmittel wie einem niederen Alkohol, beispielsweise Methanol, Ethanol, Propanol oder Isopropanol, oder einem Ether wie Diethylether oder Tetrahydrofüran oder in Dimethylformamid oder Dimethylacetamid oder in einem Gemisch der vorgenannten Lösungsmittel bei Temperaturen zwischen 0°C und dem Siedepunkt der Lösung, gewünschtenfalls in Gegenwart einer Base wie beispielsweise Natriumhydroxid oder Kaliumcarbonat oder einem Alkalialkoholat.

Reaktive Verbindungen Y-X-R₈ sind literaturbekannt oder können nach literaturbekannten Verfahren aus käuflichen Materialien hergestellt werden. 2-Amino-6-[(hydroxycarbamoyl)methyl]-azulen-1,3-dicarbonsäurediethylester ist aus EP 97110533.3 bekannt.

Bevorzugt im Sinne der vorliegenden Erfindung sind außer den in den Beispielen genannten Verbindungen und durch Kombination aller in den Ansprüchen genannten Bedeutungen der Substituenten ableitbaren Verbindungen die folgenden:
2-Amino-6-[(dimethyl-phosptunoylmethoxycarbamoyl)-methyl]-azulen-1,3-dicarbonsäure-diethylester
2-Amino-6-[(diethoxy-phosphoryloxycarbamoyl)-methyl]-azulen-1,3-dicarbonsäurediethylester
2-Amino-6-[(2,2-dimethyl-propanoyloxy)-methoxycarbamoyl-methyl]-azulen-1,3-dicarbonsäurediethylester
2-Amino-6-(ethoxymethoxycarbamoyl-methyl)-azulen-1,3-dicarbonsäurediethylester
2-Amino-6-[(3-hydroxy-propoxycarbamoyl)-methyl]-azulen-1,3-dicarbonsäurediethylester
2-Amino-6-{[2-(dimethyl-phosphinoyl)-ethoxycarbamoyl]-methyl}-azulen-1,3-dicarbonsäure- diethylester
2-Amino-6-[(1-pyrrolidin-1-yl-ethoxycarbamoyl)-methyl]-azulen-1,3-dicarbonsäurediethylester
2-Amino-6-[(1-ethoxy-1-methyl-ethoxycarbamoyl)-methyl]-azulen-1,3-dicarbonsäurediethylester
2-Amino-6-[(3-pyrrolidin-1-yl-propoxycarbamoyl)-methyl]-azulen-1,3-dicarbonsäurediethylester
2-Amino-6-[(3-hydroxy-propoxycarbamoyl)-methyl]-azulen-1,3-dicarbonsäurediethylester
2-Amino-6-{[3-(1,3-dioxo-1,3-dihydro-isoindol-2-yl)-propoxycarbamoyl]methyl}-azulen-1,3-dicarbonsäurediethylester
2-Amino-6-[(pyrrolidine-1carbonyloxycarbamoyl)-methyl]-azulen1,3-dicarbonsäurediethylester
2-Amino-6-[(2-hydroxy-ethoxycarbonylmethoxycarbamoyl)-methyl]-azulen-1,3-dicarbonsäurediethylester 2-Amino-6-(carboxymethoxycarbamoyl-methyl)-azulen-1,3-dicarbonsäurediethylester
2-Amino-6-{[(4,5-dihydro-1*H*-imidazol-2-ylcarbamoyl)-methoxycarbamoyl]-methyl}-azulen-1,3-dicarbonsäurediethylester
2-Amino-6-{[(1*H*-tetrazol-5-ylcarbamoyl)-methoxycarbamoyl]-methyl}-azulen-1,3-dicarbonsäurediethylester
2-Amino-6-(hydroxycarbamoylmethoxycarbamoyl-methyl)-azulen-1,3-dicarbonsäurediethylester
2-Amino-6-[(4-hydroxy-butoxycarbamoyl)-methyl]-azulen-1,3-dicarbonsäurediethylester
2-Amino-6-{[2-(2-hydroxyethoxy)-ethoxycarbamoyl]-methyl}-azulen-1,3-dicarbonsäurediethylester
2-Amino-6-{[2-(1,3-dioxo-1,3-dihydro-isoindol-2-yl)-ethoxycarbamoyl]-methyl}-azulen-1,3-dicarbonsäurediethylester
2-Amino-6-{[(dimethyl-phosphinoylmethoxy)-methyl-carbamoyl]-methyl}-azulen-1,3-dicarbonsäurediethylester
2-Amino-6-[(methyl-phosphonomethoxy-carbamoyl)-methyl]-azulen-1,3-dicarbonsäurediethylester
2-Amino-6-{[(diethoxy-phosphorylmethoxy)-methyl-carbamoyl]-methyl}-azulen-1,3-dicarbonsäurediethylester
2-Amino-6-{[methyl-(3-pyrrolidin-1-yl-propoxy)-carbamoyl]methyl}-azulen-1,3-dicarbonsäurediethylester
2-Amino-6-({[3-(1,3-dioxo-1,3-dihydro-isoindol-2-yl)-propoxy]-methyl-carbamoyl}-methyl)-azulen-1,3-dicarbonsäurediethylester
2-Amino-6-{[methyl-(2-oxo-2-pyrrolidin-1-yl-ethoxy)-carbamoyl]-methyl}-azulen-1,3-dicarbonsäurediethylester
2-Amino-6-[(ethoxycarbonylmethoxy-methyl-carbamoyl)-methyl]-azulen-1,3-dicarbonsäurediethylester
2-Amino-6-[(carboxymethoxy-methyl-carbamoyl)-methyl]-azulen-1,3-dicarbonsäurediethylester
2-Amino-6-[(hydroxycarbamoylmethoxy-methyl-carbamoyl)-methyl]-azulen-1,3-dicarbonsäurediethylester
2-Amino-6-{[(2-ethoxy-ethoxy)-methyl-carbamoyl]-methyl}-azulen-1,3-dicarbonsäurediethylester
2-Amino-6-({[2-(2-hydroxy-ethoxy)-ethoxy]-methyl-carbamoyl}-methyl)-azulen-1,3-dicarbonsäurediethylester
2-Amino-6-{[(2-hydroxy-ethoxy)-methyl-carbamoyl]-methyl}-azulen-1,3-dicarbonsäurediethylester
2-Amino-6-({[2-(1,3-dioxo-1,3-dihydro-isoindol-2-yl)-ethoxy]-methyl-carbamoyl}-methyl)-azulen-1,3-dicarbonsäurediethylester
2-Amino-6-{[ethyl-(2-oxo-2-pyrrolidin-1-yl-ethoxy)-carbamoyl]-methyl}-azulen-1,3-dicarbonsäurediethylester
2-Amino-6-({[(4,5-dihydro-1*H*-imidazol-2-ylcarbamoyl)-methoxy]-ethyl-carbamoyl}-methyl)-azulen-1,3-dicarbonsäurediethylester
2-Amino-6-({[2-(1,3-dioxo-1,3-dihydro-isoindol-2-yl)-ethoxy]-ethyl-carbamoyl}-methyl)-azulen-1,3-dicarbonsäurediethylester

Die folgenden Beispiele legen die Erfindung exemplarisch dar. ohne sie jedoch auf diese einzuschränken:

### Beispiel 1

### 2-Amino-6-[(2-oxo-2-piperidin-1yl-ethoxycarbamoyl)-methyl]azulen-1,3-dicarbonsäurediethylester

Eine Mischung aus 0.96 g (2.5 mmol) 2-Amino-6-[(hydroxycarbamoyl)-methyl]-azulen-1,3-dicarbonsäurediethylester-natrium, 50 ml Methanol, 0.4 g (2.5 mmol) N-Chloracetylpiperidin und 20 mg Natriumiodid wird 20 Stunden am Rückfluß erhitzt. Danach engt man ein und chromatographiert an Kieselgel. Mit Isohexan:Ethylacetat 1:1 eluiert man 0.42 g (35% d.Th.) Titelverbindung vom Schmp. 153 - 155 °C.

### Beispiel 2

### 2-Amino-6-[(2-oxo-2-pyrrolidin-1-yl-ethoxycarbamoyl)-methyl]-azulen-1,3-dicarbonsäurediethylester

In analoger Weise wie in Beispiel 1 beschrieben erhält man aus 2-Amino-6-[(hydroxycarbamoyl)-methyl]-azulen-1,3-dicarbonsäurediethylester-natrium und N-Chloracetyl-pyrrolidin in 24% Ausbeute die Titelverbindung vom Schmp. 168 - 170 °C.

### Beispiel 3

### 2-Amino-6-[(2-oxo-2-morpholin-4-yl-ethoxycarbamoyl)-methyl]-azulen-1,3-dicarbonsäurediethylester

In analoger Weise wie in Beispiel 1 beschrieben erhält man aus 2-Amino-6-[(hydroxycarbamoyl)-methyl]-azulen-1,3-dicarbonsäurediethylester-natrium und N-Chloracetyl-morpholin in 33% Ausbeute die Titelverbindung vom Schmp. 196 - 198 °C.

### Beispiel 4

### 2-Amino-6-{[2-oxo-2-(N,N-bis-2-methoxyethyl-amino)-ethoxycarbamoyl]-methyl}-azulen-1,3-dicarbonsäurediethylester

In analoger Weise wie in Beispiel 1 beschrieben erhält man aus 2-Amino-6-[(hydroxycarbamoyl)-methyl]-azulen-1,3-dicarbonsäurediethylester-natrium und N-Chloracetyl-N,N-bis-2-methoxyethyl-amin in 31% Ausbeute die Titelverbindung vom Schmp 109 - 112 °C.

### Beispiel 5

### 2-Amino-6-[(2-oxo-2-N,N-dimethylamino-ethoxycarbamoyl)-methyl]-azulen-1,3-dicarbonsäurediethylester

In analoger Weise wie in Beispiel 1 beschrieben erhält man aus 2-Amino-6-[(hydroxycarbamoyl)-methyl]-azulen-1,3-dicarbonsäurediethylester-natrium und 2-Chlor-N,N-dimethylacetamid in 27% Ausbeute die Titelverbindung vom Schmp. 177 - 179 °C.

### Beispiel 6

### 2-Amino-6-[(2-oxo-2-ethoxy-ethoxycarbamoyl)-methyl]-azulen-1,3-dicarbonsäurediethylester

In analoger Weise wie in Beispiel 1 beschrieben erhält man aus 2-Amino-6-[(hydroxycarbamoyl)-methyl]-azulen-1,3-dicarbonsäurediethylester-natrium und Bromessigsäureethylester in 24% Ausbeute die Titelverbindung als Wachs.

### Beispiel 7

### 2-Amino-6-[(2-hydroxy-ethoxycarbamoyl)-methyl]-azulen-1,3-dicarbonsäurediethylester

In analoger Weise wie in Beispiel 1 beschrieben erhält man aus 2-Amino-6-[(hydroxycarbamoyl)-methyl]-azulen-1,3-dicarbonsäurediethylester-natrium und 2-Bromethanol in 15% Ausbeute die Titelverbindung vom Schmp. 190 - 192 °C.

### Beispiel 8

### 2-Amino-6-[(2,2-dimethyl-1,3-dioxolan-4-ylmethoxycarbamoyl)-methyl]azulen-1,3-dicarbonsäurediethylester

In analoger Weise wie in Beispiel 1 beschrieben erhält man aus 2-Amino-6-[(hydroxycarbamoyl)-methyl]-azulen-1,3-dicarbonsäurediethylester-natrium und 2,2-Dimethyl-4-(4-methyl-benzolsulfonyloxy-methyl)-1,3-dioxolan in 15% Ausbeute die Titelverbindung vom Schmp. 135 - 137 °C.

### Beispiel 9

### 2-Amino-6-[(2,3-dihydroxy-propoxycarbamoyl)-methyl]-azulen-1,3-dicarbonsäurediethylester

Durch Umsetzung der in Beispiel 8 beschriebenen Verbindung mit verd. Salzsäure und anschließende chromatographische Reinigung erhält man in 13% Ausbeute die Titelverbindung vom Schmp. 151 - 153 °C.

### Beispiel 10

### 2-Amino-6-[(2-hydroxy-3-methoxy-propoxycarbamoyl)-methyl]-azulen-1,3-dicarbonsäurediethylester

In analoger Weise wie in Beispiel 1 beschrieben erhält man aus 2-Amino-6-[(hydroxycarbamoyl)-methyl]-azulen-1,3-dicarbonsäurediethylester-natrium und 2-Methoxymethyl-oxiran in 15% Ausbeute die Titelverbindung als Wachs.

### Beispiel 11

### 2-Amino-6-{[2-oxo-2-(N,N-bis-2-hydroxyethylamino)-ethoxycarbamoyl]-methyl}-azulen-1,3-dicarbonsäurediethylester

In analoger Weise wie in Beispiel 1 beschrieben erhält man aus 2-Amino-6-[(hydroxycarbamoyl)-methyl]-azulen-1,3-dicarbonsäurediethylester-natrium und N-Chloracetyl-N,N-bis-2-hydroxyethyl-amin in 23% Ausbeute die Titelverbindung als Wachs.

### Beispiel 12

### 2-Amino-6-{[(N,N-dimethylamino)-carbonyloxy-carbamoyl]-methyl}-azulen-1,3-dicarbonsäurediethylester

In analoger Weise wie in Beispiel 1 beschrieben, jedoch unter Verwendung von N,N-Dimethylformamid anstelle von Methanol, erhält man aus 2-Amino-6-[(hydroxycarbamoyl)-methyl]-azulen-1,3-dicarbonsäurediethylester-natrium und Chlorameisensäuredimethylamid in 17% Ausbeute die Titelverbindung vom Schmp 167 - 169 °C.

### Beispiel 13

### 2-Amino-6-{[2-oxo-2-(N,N-bis-2-methoxyethyl-amino)-N-methyl-ethoxycarbamoyl]-methyl}-azulen-1,3-dicarbonsäurediethylester

In analoger Weise wie in Beispiel 1 beschrieben erhält man aus 2-Amino-6-[(N-methylhydroxycarbamoyl)-methyl]-azulen-1,3-dicarbonsäurediethylester-natrium und N-Chloracetyl-N,N-bis-2-methoxyethyl-amin in 60% Ausbeute die Titelverbindung als Wachs.

### Beispiel 14

### 2-Amino-6-[(2-piperidin-1-yl-ethoxycarbamoyl)-methyl]-azulen-1,3-dicarbonsäurediethylester

In analoger Weise wie in Beispiel 1 beschrieben erhält man aus 2-Amino-6-[(hydroxycarbamoyl)-methyl]-azulen-1,3-dicarbonsäurediethylester-natrium und 2-Piperidino-ethylbromid in 7% Ausbeute die Titelverbindung vom Schmp. 163 - 165 °C

### Beispiel 15

### 2-Amino-6-[(3-dimethylamino-2-hydroxy-propoxycarbamoyl)-methyl]-azulen-1,3-dicarbonsäurediethylester

Eine Mischung aus 1.14 g (3 mmol) 2-Amino-6-[(hydroxycarbamoyl)-methyl]-azulen-1,3-dicarbonsäurediethylester-natrium, 50 ml Ethanol, 0.1 ml Triethylamin und 0.25 ml Epichlorhydrin wird 20 h zum Rückfluß erwärmt, anschließend eingeengt und an Kieselgel chromatographiert. Mit Isohexan:Ethylacetat 1:1 isoliert man 0.2 g des gewünschten Epoxids, das mit überschüssiger ethanolischer Dimethylaminlösung versetzt wird. Nach einem Tag Stehen bei Raumtemperatur wird an Kieselgel chromatographiert. Mit Isohexan:Ethylacetat 1:1 isoliert man 60 mg (4% d.Th.) der Titelverbindung als Wachs.

### Beispiel 16

### 2-Amino-6-[(diethoxy-phosphorylmethoxycarbamoyl)-methyl]-azulen-1,3-dicarbonsäure- diethylester

Eine Lösung von 2.5 mmol 2-Amino-6-carboxymethyl-azulen-1,3-dicarbonsäurediethylester in 50 ml Tetrahydrofuran wird bei 45 °C portionsweise mit 2.5 mmol N,N'-Carbonyldiimidazol versetzt. Man rührt 30 min nach, fügt zu der erhaltenen Mischung eine Lösung von 2.5 mmol Diethoxyphosphorylmethoxyamin (L. Maier, Phosphorus, Sulfur, and Silicon 1993, Vol. 76, 119-122) in 20 ml Tetrahydroffiran und erwärmt 3 h zum Rückfluß. Man engt ein und chromatographiert an Kieselgel. Mit Isohexan:Ethylacetat 1:1 eluiert man 78% der Titelverbindung vom Schmp. 146 - 147 °C.

### Beispiel 17

### 2-Amino-6-(phosphonomethoxycarbamoyl-methyl)-azulen-1,3-dicarbonsäurediethylester

In analoger Weise wie in Beispiel 1 beschrieben erhält man aus 2-Amino-6-carboxymethyl-azulen-1,3-dicarbonsäurediethylester und Phosphonomethoxyamin (L. Maier, Phosphorus, Sulfur, and Silicon 1993, Vol. 76, 119-122) die Titelverbindung als amorphes Pulver.

### Beispiel 18

### 2-Amino-6-[(2-oxo-2-pyrrolidin-1-yl-N-methyl-ethoxycarbamoyl)-methyl]-azulen-1,3-dicarbonsäurediethylester

In analoger Weise wie in Beispiel 1 beschrieben erhält man aus 2-Amino-6-[(N-methylhydroxycarbamoyl)-methyl]-azulen-1,3-dicarbonsäurediethylester-natrium und N-Chloracetyl-pyrrolidin mit 77% Ausbeute die Titelverbindung als Wachs.

## Patentansprüche

1. Azulenderivate der allgemeinen Formel I, 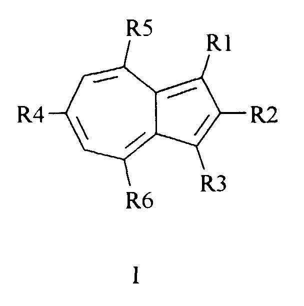 wobei
R₁ und R₃
einzeln und unabhängig voneinander Wasserstoff Hydroxy, Amino, Mercapto, einen geraden oder verzweigten, gesättigten oder ungesättigten aliphatischen Rest mit 1 - 15 Kohlenstoffatomen, der ein oder mehrfach substituiert sein kann, eine Alkoxy-, Alkylamino-, Dialkylamino-, Alkylmercapto-, Alkylsulfinyl-, Alkylsulfonyl-, Alkenyl-, Alkinyl-, Alkenyloxy-, Alkenylmercapto-, Alkinyloxy-, Alkinylmercapto-, Alkylcarbonylamino-, Dialkylcarbonylamino-, Alkylaminocarbonyl-, Formyl-, Alkylcarbonyl-, Carboxyl-, Alkoxycarbonyl-, Alkenyloxycarbonyl-, Alkinyloxycarbonyl-, Carboxyalkyl-, Alkyloxycarbonylalkyl, Alkenyloxycarbonylalkyl-, Alkinyloxycarbonylalkyl-, Nitro-, Cyano-, Halogen-, Trifluormethyl-, oder Azidogruppe, Phenyl oder Phenylcarbonyl, das gegebenenfalls ein- oder mehrfach unabhängig voneinander substituiert sein kann, einen carbocyclischen mono-, bi- oder tricyclischen Rest mit 7-15 C-Atomen oder ein heterocyclisches mono-, bi- oder tricyclisches Ringsystem bedeuten.
R₂
Wasserstoff, Hydroxy, Amino, Mercapto, einen geraden oder verzweigten, gesättigten oder ungesättigten aliphatischen Rest mit 1 - 15 Kohlenstoffatomen, der ein- oder mehrfach substituiert sein kann, eine Alkoxy-, Alkylamino-, Dialkylamino-, Alkylmercapto-, Alkylsulfinyl-, Alkylsulfonyl-, Alkenyl-, Alkinyl-, Alkenyloxy-, Alkenylmercapto-, Alkinyloxy-, Alkinylmercapto-, Alkylcarbonylamino-, Dialkylcarbonylamino-, Alkylaminocarbonyl-, Formyl-, Alkylcarbonyl-, Carboxyl-, Alkoxycarbonyl-, Alkenyloxycarbonyl-, Alkinyloxycarbonyl-, Carboxyalkyl-, Alkyloxycarbonylalkyl, Alkenyloxycarbonylalkyl-, Alkinyloxycarbonylalkyl-, Nitro-, Cyano-, Halogen-, Trifluormethyl-, oder Azidogruppe, Phenyl oder Phenylcarbonyl, das gegebenenfalls ein- oder mehrfach unabhängig voneinander substituiert sein kann, einen carbocyclischen mono-, bi- oder tricyclischen Rest mit 7-15 C-Atomen oder ein heterocyclisches mono-, bi- oder tricyclisches Ringsystem bedeutet,
R₄
eine Gruppe CH₂CON(R₇)OXR₈ darstellt,
R₅ und R₆
einzeln und unabhängig voneinander Wasserstoff einen geraden oder verzweigten, gesättigten oder ungesättigten aliphatischen Rest mit 1 - 15 Kohlenstoffatomen, der ein- oder mehrfach substituiert sein kann, eine Alkoxy-, Alkylamino-, Dialkylamino-, Alkylmercapto-, Alkylsulfinyl-, Alkylsulfonyl-, Alkenyl-, Alkinyl-, Alkenyloxy-, Alkenylmercapto-, Alkinyloxy-, Alkinylmercapto-, Alkylcarbonylamino-, Dialkylcarbonylamino-, Alkylaminocarbonyl-, Formyl-, Alkylcarbonyl-, Carboxyl-, Alkoxycarbonyl-, Alkenyloxycarbonyl-, Alkinyloxycarbonyl-, Carboxyalkyl-, Alkyloxycarbonylalkyl, Alkenyloxycarbonylalkyl-, Alkinyloxycarbonylalkyl-, Nitro-, Cyano-, Halogen-, Trifluormethyl-, oder Azidogruppe, Phenyl oder Phenylcarbonyl, das gegebenenfalls ein oder mehrfach unabhängig voneinander substituiert sein kann, einen carbocyclischen mono-, bi- oder tricyclischen Rest mit 7-15 C-Atomen oder ein heterocyclisches mono-, bi- oder tricyclisches Ringsystem,
R₇
Wasserstoff oder einen Alkylrest,
X
eine Valenzbindung, eine Alkylengruppe, eine Carbonylgruppe oder eine Alkylencarbonylgruppe,
R₈
eine Alkylgruppe, die gewünschtenfalls ein- oder mehrfach gleich oder verschieden durch Hydroxy, Alkoxy, Amino, Alkylamino oder Dialkylamino substituiert sein kann, eine gewünschtenfalls in 2-Stellung mono- oder disubstituierte 1,3-Dioxolan-4-ylgruppe oder eine Gruppe PO(R₁₁)(R₁₂) oder PO(OR₁₃)(OR₁₄)
sowie für den Fall, daß X nicht eine Valenzbindung darstellt, auch eine Hydroxy-, Alkoxy-, Hydroxyalkoxy, Alkanoyloxy-, Alkylcarbonylamino-, Alkoxycarbonyloxy-, Alkanoyloxyalkyl-, Carboxyvinylgruppe, eine gewünschtenfalls substituierte Aminoalkanoyloxy-, Aminoalkylphenyl- oder Aminoalkylbenzoyloxygruppe, eine Alkanoyloxyalkoxygruppe oder eine Gruppe NR₉R₁₀,
R₉ und R₁₀
gleich oder verschieden Wasserstoff, einen Alkylrest, der gewünschtenfalls ein- oder mehrfach gleich oder verschieden durch Hvdroxy, Alkoxy, Amino, Alkylamino oder Dialkylamino substituiert sein kann,
oder R₉ Wasserstoff und R₁₀ Hydroxy, Imidazolin-2-yl oder Tetrazol-5-yl bedeuten oder R₉ und R₁₀ gemeinsam mit dem Stickstoffatom, an das sie gebunden sind, einen heterocyclischen Ring bilden, der gewünschtenfalls durch Stickstoff oder Sauerstoff unterbrochen und durch Alkyl, Alkoxy, Hydroxy, Hydroxyalkyl, Oxo, Carboxy, Aminocarbonyl oder Alkoxycarbonyl substituiert und mit einem benzoiden Ring anelliert sein kann,
R₁₁ und R₁₂
gleich oder verschieden sind und einen Alkylrest und
R₁₃ und R₁₄
gleich oder verschieden sein können und Wasserstoff oder einen Alkylrest darstellen
sowie deren Tautomere, Enantiomere, Diastereomere, Racemate und physiologisch verträgliche Salze oder Ester und Substanzen, die *in viv*o zu Verbindungen der Formel I hydrolysiert oder metabolisiert werden.

2. Arzneimittel, enthaltend mindestens eine Verbindung der allgemeinen Formel I gemäß Anspruch 1 neben üblichen pharmazeutischen Hilfs- und Trägerstoffen.

3. Verwendung von Verbindungen der Formel I gemäß Anspruch 1 oder 2 zur Herstellung von Arzneimitteln mit Metalloprotease-inhibierender Wirkung.

4. Pharmazeutische Zubereitung, dadurch gekennzeichnet, daß sie ein Arzneimittel gemaß Anspruch 2 enthält.
